# FASCICULE DE BREVET EUROPEEN

(11) **EP 3 201 282 B1**
(45) Date de publication et mention de la délivrance du brevet: **04.03.2026**
(21) Numéro de dépôt: 15785060.3
(22) Date de dépôt: 02.10.2015
(51) Int. Cl.: C09J 5/02, C40B 50/18, B82Y 40/00

(54) **IMPRESSION D'UN MOTIF ADHESIF SUR UN SUPPORT ANTI-SALISSURES**
DRUCKEN EINES KLEBEMUSTERS AUF EINEN FÄULNISVERHINDERNDEN TRÄGER
PRINTING AN ADHESIVE PATTERN ON AN ANTI-FOULING SUPPORT

(30) Priorité: 03.10.2014 FR 1459497
(43) Date de publication de la demande: 09.08.2017
(73) Titulaire: Alveole, 75005 Paris (FR); Centre National de la Recherche Scientifique (C.N.R.S.), 75794 Paris Cedex 16 (FR); Université de Bordeaux, 33076 Bordeaux (FR)
(72) Inventeur: STUDER, Vincent, F-33000 Bordeaux (FR)
(74) Mandataire: Osha BWB
(86) Numéro de dépôt international: PCT/EP2015/072874
(87) Numéro de publication internationale: WO 2016/050980

(56) Documents cités:
- WO-A1-2006/084482
- WO-A1-2013/135844
- SERGE COSNIER ET AL: "An electrogenerated poly(pyrrole-benzophenone) film for the photografting of proteins", CHEMICAL COMMUNICATIONS, no. 3, 23 January 2003 (2003-01-23), pages 414 - 415, XP055107603, ISSN: 1359-7345, DOI: 10.1039/b211546h
- D. LECKBAND , S. SHETH & A. HALPERIN, JOURNAL OF BIOMATERIALS SCIENCE, POLYMER EDITION, 2 April 2012 (2012-04-02), pages 1125 - 1147, XP002738831, Retrieved from the Internet <URL:http://www.tandfonline.com/doi/pdf/10.1163/156856299X00720> [retrieved on 20150421]

## Description

### DOMAINE TECHNIQUE

La présente invention concerne le domaine du greffage d'une protéine sur un substrat, selon un motif défini optiquement.

### ARRIERE PLAN

La publication de la demande internationale de numéro WO2013/135844 (ci-après « STUDER » ou « la publication ») divulgue un dispositif de greffage micro-structuré de protéines sur un substrat ou d'impression photochimique. Dans la publication, un mélange en solution aqueuse d'une benzophénone (BP) et d'une protéine est illuminé par endroits selon un motif au-dessus d'un substrat et un transfert durable de la protéine aux endroits éclairés est obtenu, réalisant l'impression. Toutefois, le procédé décrit dans la publication effectue un transfert de la protéine sur le substrat, en présence de BP et de façon simultanée à l'éclairement. Ce dispositif implique une combinaison au même endroit d'un dispositif d'éclairement selon une image d'un motif sur le substrat et d'un dispositif micro-fluidique permettant d'amener une solution aqueuse contenant simultanément une protéine et une BP. Il en résulte un problème d'encombrement du système d'impression ainsi qu'un risque d'endommager la protéine par l'action combinée de la benzophénone et de la lumière du dispositif d'éclairement.

D'autres exemples de procédés photochimiques de greffage de protéines sur un substrat sont décrits dans le document de brevet WO 2006/084482 et dans la publication scientifique "An electrogenerated poly(pyrrole-benzophenone) film for the photografting of proteins" de Cosnier et al.

Idéalement, il serait utile d'imprimer un motif simplement adhésif pour la protéine, sans protéine adhérée, sur un substrat, au moyen du système d'éclairage. Un motif réel se révélerait postérieurement sur le substrat, au contact d'une solution aqueuse d'une protéine, par exemple fluorescente, la protéine s'accrochant préférentiellement aux parties illuminées dans le motif adhésif pour former le motif réel. Une telle solution est néanmoins conditionnée, pour une protéine, par la mise à disposition d'un procédé apte à produire un motif adhésif latent ou révélé ultérieurement, qui soit imprimé sur un support recouvert d'une couche anti-salissures (en anglais : "antifouling") pour les protéines. Par couche anti-salissures pour les protéines, on entend désigner une couche réalisée dans un matériau ne présentant pas d'accrochage des protéines sur ladite couche, à l'échelle de temps de la réalisation de l'impression qu'on se propose de réaliser.

Un tel substrat recouvert de sa couche anti-salissures ou substrat anti-salissures peut être constitué notamment par un support comme un support rigide, dont un exemple est un verre de qualité optique, transparent pour la lumière du système d'éclairage ou comme un support mou dont un exemple est un PDMS, le verre ou le PDMS étant recouverts d'un matériau à brosse de polymère, ou polymère s'accrochant en brosse au support par chaînes de molécules, comme le PEG et le polyNipam. Les chaînes de polymère sont pour des substrats anti-salissures de ce type, accrochées à une de leurs extrémités au support et libres à l'autre extrémité comme les poils d'une brosse.

D'autres techniques comme la photolithographie appliquée à un substrat anti-salissures pour les protéines au travers d'un masque utilisant l'ablation par laser de motifs de matériaux anti-salissures sur un substrat anti-salissures, permettent dans l'art antérieur d'obtenir des supports anti-salissures présentant des motifs permettant le greffage sélectif ultérieur d'une protéine sur le substrat, selon les zones éclairées du substrat desquelles ont été enlevées, par l'énergie lumineuse, le matériau anti-salissures ou brosse de polymère.

Il est estimé que l'ablation de matière est occasionnée par l'éclairage du substrat et que les différences de niveau produites permettent une image en creux de l'image réelle ultérieure. Lorsque ces différences sont observéespar la technique optique du contraste de phase, qui n'est sensible qu'au chemin optique, le motif adhésif peut être attribué de façon équivalente à une ablation de matière anti-salissures ou à un changement de nature du matériau modifiant son indice optique et procurant une adhésion préférentielle ultérieure des protéines sur les zones de chaînes de polymères ayant été éclairées. D'autres techniques qui permettent d'observer l'image latente (notamment microscopie par force atomique, ellipsométrie, Analyse X, etc...) permettent dans certains cas de prouver que l'image latente est due à une ablation totale de la couche de PEG pour ces techniques. De telles techniques d'ablation ne permettent donc pas de réaliser des gradients de concentration, l'ablation de la couche de PEG ou anti-salissures étant à priori totale.

Il serait finalement souhaitable de disposer d'un procédé pour produire un support anti-salissures ou à brosse de polymère présentant une adhésion proportionnelle ou continûment variable avec l'exposition de la brosse à une illumination, selon un motif, sans que des molécules soient nécessairement adhérées à la brosse simultanément à l'illumination. Il serait plutôt souhaitable que ces molécules soient adhérées à la brosse de façon différée.

### PRESENTATION GENERALE

Les définitions suivante s'appliquent à la présente demande :
"Motif adhésif" (en anglais "adhesive pattern") : désigne un motif surfacique selon lequel certaines molécules, notamment des protéines (et surtout des anticorps), des nano-billes, des brins d'ADN (acide désoxyribonucléique) ou d'ARN ou des bactéries se répartissent de façon stable dans le temps sur un support recouvert par une couche anti-adhésive ou anti-salissures ou à brosse de polymère, en dehors dudit motif adhésif. Le motif étant défini en dehors d'une zone ou un ensemble de zones anti-adhésives ou anti-salissures (en anglais "antifouling" ou "anti-fouling"), un motif adhésif peut aussi être défini sur un substrat comme un ensemble de zones ou motifs qui sont plus adhésives pour les molécules d'intérêt, que la surface complémentaire de l'ensemble des zones sur le substrat. Une différence d'effet d'adhésion, nécessaire à l'existence d'un motif, peut être prédite pour une brosse de polymère, sans mise en contact avec une solution aqueuse d'une molécule, par au moins deux techniques disponibles dans l'art antérieur:
- la microscopie à force atomique qui permet de mettre en évidence une réduction de longueur des chaînes de polymère de la brosse, une telle réduction causant alors une réduction de l'effet anti-adhésif ou une augmentation de l'effet d'adhésion dans ces zones.
- La microscopie en contraste de phase, qui permet de mettre en évidence une variation de chemin optique à travers la brosse dans les zones plus adhésives, une telle variation étant alors associée à une variation d'effet d'adhésion.

"Brosse de polymère" : désigne une couche nanométrique (c'est à dire dont l'épaisseur est à l'échelle nanométrique, soit typiquement entre 1nm et 100 nm) et anti-salissures, notamment pour les protéines, les nano-billes, les brins d'ADN et les bactéries, une telle couche nanométrique étant présente à la surface d'un support pour former un substrat anti-salissures. Il est estimée, à la date de la présente demande, qu'une telle brosse est constituée d'un ensemble de chaînes de polymère greffées à la surface d'un support, cet ensemble s'étendant dans une zone d'épaisseur comprise entre 1nm et 20nm à la surface du support pour du PEG et entre 1nm et 30nm pour du polyNIPAM. Il est estimé qu'entre 1nm et 20nm, une telle brosse possède des propriétés d'anti-adhésion ou d'anti-salissure ou d'anti-salissures, notamment pour les protéines, les nano-billes, les brins d'ADN ou les bactéries. Une couche de polyéthylèneglycol ou "PEG" ou une couche de poly(N-isopropylacrylamide) ou polyNIPAM sont des exemples de brosses de polymère.

"Epaisseur" désigne pour une brosse de polymère la mesure de la distance au support des extrémités libres des chaînes de polymère constituant la brosse. Par exemple, pour du PEG, l'épaisseur de la couche est contrôlée par la longueur des chaines de PEG, c'est à dire le nombre de monomères d'Ethylène Glycol composant ces chaines. Ces chaines peuvent être notamment inclinées par rapport au substrat ou tassées ou modifiées de toute manière semblable à une action sur les poils d'une brosse pour imprimer à la surface libre de la brosse un relief ou une variation d'épaisseur.

Dans ce contexte, l'invention concerne un procédé pour imprimer un motif adhésif, sur une brosse d'un polymère s'étendant à la surface d'un support en une couche nanométrique anti-salissures, tel que défini dans la revendication 1.

Dans des variantes du procédé :
- l'épaisseur de la couche est comprise entre 1nm et 20nm.
- la longueur d'onde est choisie entre 300nm et 400nm.
- le polymère est un polyéthylène glycol (PEG).
- le polymère est un polyNIPAM.
- le support est un verre.
- le support est un PolyDiMethylSiloxane (PDMS).
- l'énergie surfacique de l'éclairage communiquée à la couche de PEG est comprise entre 10mJ/mm2 et 1000mJ/mm2.
- l'énergie surfacique de l'éclairage communiquée à la couche de polyNIPAM est comprise entre 100mJ/mm2 et 10000mJ/mm2.
- le module d'Young du support en PDMS est inférieur à 15kPa.

L'invention concerne aussi un procédé comme ci-dessus, pour imprimer un motif d'une protéine sur la brosse du polymère, comprenant les étapes supplémentaires suivantes :
- rincer pour supprimer le contact entre la couche et la première solution.
- puis mettre la couche en contact avec une seconde solution aqueuse contenant la protéine. L'invention concerne aussi un procédé comme ci-dessus, pour imprimer un motif de nano-billes sur la brosse du polymère, comprenant les étapes supplémentaires suivantes :
- rincer pour supprimer le contact entre la couche et la première solution
- mettre la couche en contact avec une seconde solution contenant les nano-billes.

L'invention concerne aussi un procédé comme ci-dessus, pour imprimer un motif de brins d'ADN sur la brosse du polymère, comprenant les étapes supplémentaires suivantes :
- rincer pour supprimer le contact entre la couche et la première solution
- mettre la couche en contact avec une seconde solution contenant les brins d'ADN.

L'invention concerne aussi une application du procédé pour imprimer un motif adhésif, à la réalisation d'un motif adhésif présentant un gradient d'adhésion à la surface du support, par variation spatiale de l'énergie surfacique.

### BREVE DESCRIPTION DES DESSINS

L'invention sera mieux comprise en lien avec la liste des figures ci-dessous dans laquelle :

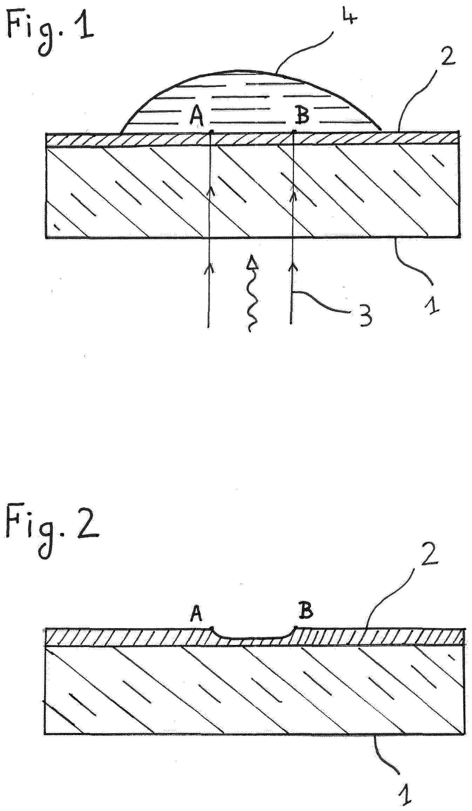

- La figure 1 représente en coupe, un substrat anti-salissures composé d'un support en verre et d'une couche de matériau anti-salissures notamment pour les protéines, les nano-billes ou les brins d'ADN, c'est-à-dire une brosse de polymère greffée ou accrochée au support. Le substrat est recouvert d'une goutte d'une solution aqueuse contenant de la benzophénone sur tout ou partie de la brosse de polymère. Une zone AB de la couche, recouverte de benzophénone, est illuminée à travers le support (à travers la goutte serait aussi praticable) par un rayonnement comprenant des longueurs d'onde dans le spectre d'absorption de la benzophénone soit un rayonnement entre 300nm et 400nm.
- La figure 2 représente le substrat rincé de la goutte de la figure 1 et présentant une image latente représentée de façon imagée par un creux dans la surface du matériau anti-salissures au niveau de la zone AB. Un tel matériau est apte à être utilisé pour imprimer notamment un motif d'une protéine, de nano-billes ou des brins d'ADN ou d'ARN selon un motif correspondant à la surface de la zone AB. En effet, l'illumination de la brosse de polymère en présence de benzophénone, rend potentiellement adhésive la brosse de polymère dans la zone AB et permet l'adhésion ultérieure notamment d'une protéine, de nano-billes ou de brins d'ADN, en mettant en contact ultérieurement la couche de brosse de polymère, respectivement avec notamment une solution de la protéine, une solution de nano-billes ou de brins d'ADN. Une illumination selon un ensemble de zones comme AB permet ainsi la réalisation d'un motif adhésif ou un motif adhésif de molécules sur une brosse de polymère, pour des molécules pour lesquelles la brosse est normalement non-adhésive.

### DESCRIPTION DETAILLEE D'EXEMPLE(S)

Dans un premier mode de réalisation, il est divulgué en référence à la figure 1 pour les numéros de référence entre parenthèses, un substrat anti-salissures composé d'un support en verre (1) et d'une couche (2) d'un matériau à brosse de polymère (en anglais : "polymer brush") qui est, dans ce premier mode, du PEG ou du polyNIPAM.

Dans ce premier mode, un rayonnement (3) éclaire la couche (2) selon une zone AB (AB), ici à travers un support (1) choisi transparent pour le rayonnement utilisé, une goutte (4) d'une solution aqueuse de benzophénone est déposée sur la couche (2) en recouvrant la zone AB (AB). De façon équivalente, il serait possible d'éclairer la couche à travers la goutte (4), selon la même zone AB.

Le rayonnement utilisé comporte au moins une longueur d'onde comprise dans le spectre d'absorption de la benzophénone, spectre qui s'étend utilement en pratique, entre 300nm et 400nm. Préférentiellement, on utilisera dans cet intervalle un rayonnement de longueur d'onde inférieure à 390nm, dans ce cas, le temps d'exposition de la couche au rayonnement sera minimisé.

Moins il y aura d'absorption de la benzophénone à la longueur d'onde choisie et plus la puissance de la source lumineuse devra être importante, ou plus le temps d'exposition de la zone éclairée devra être long, la dose de radiation reçue, égale au produit de la puissance lumineuse par le temps d'exposition à la lumière, étant le paramètre gouvernant l'obtention de l'effet de l'invention.

Aucune protéine à greffer n'étant en solution, le rayonnement sera si besoin de puissance supérieure à une puissance provoquant la destruction d'une protéine à greffer ultérieurement et ne sera limité que par la densité surfacique d'énergie lumineuse acceptée par la couche, sans dégradation. Toutefois, la présence de benzophénone permet pour du PEG d'utiliser des puissances optiques 10 à 100 fois moindres que pour des techniques d'ablation ou de masquage.

Une densité d'énergie comprise entre 10mJ/mm2 et 1000mJ/mm2 est ainsi utilisable pour obtenir l'apparition d'un motif adhésif sur du PEG. L'invention peut ainsi se contenter d'une source produisant un éclairement de 2mW sur un carré de 400 microns de côté pour une longueur d'onde d'une raie ultraviolette à 372nm d'un laser à semi-conducteur. Pour du polyNipam sur un support de PDMS, une densité d'énergie utilisable est comprise entre 100mJ/mm2 et 10000mJ/mm2. La même source laser à semi-conducteur peut encore être utilisée en multipliant simplement les temps d'exposition pour du PEG par 10.

Dans une première étape du procédé de ce mode de réalisation, on met en contact le substrat anti-salissures avec une goutte de solution aqueuse de benzophénone, puis dans une deuxième étape on éclaire une zone AB de la couche anti-salissures du substrat avec la source lumineuse ultraviolette.

Un système optique quelconque permettant la focalisation de l'énergie de la source sur la zone AB ou sur un ensemble de zones en même temps est utilisable et de tels systèmes sont connus de l'art antérieur. Un microscope avec une matrice de micro-miroirs est ainsi envisageable pour réaliser le système d'éclairage pour ce mode de réalisation. De même, la goutte peut être remplacée par un film de solution aqueuse de benzophénone amené en contact avec la couche puis rincé après éclairement par des moyens micro-fluidiques connus.

La figure 2 représente la brosse de polymère formée en couche nanométrique, ainsi rincée de la goutte de la solution de benzophénone et munie de façon imagée, d'un motif latent, en creux lui aussi nanométrique quant à sa profondeur, au niveau de la zone AB. Ce motif en creux ou latent nécessite pour être révélé d'être mis ultérieurement en contact avec des molécules ou des ensembles moléculaires susceptibles d'adhérer au support au niveau de ce creux dans une brosse de polymère (protéines, nano-billes, brins d'ADN, bactéries, ...), l'adhésion de ces molécules selon le motif latent se produit alors, en présence de ces molécules en solution aqueuse, au niveau des zones de la couche qui ont été éclairées (ici AB) en présence de benzophénone. L'adhésion de molécules se fait sans apport d'énergie lumineuse. Les molécules sont simplement adsorbées sur la brosse de polymère au niveau du motif latent ou motif adhésif. Il se forme ainsi un motif réel de molécules sur la brosse. Notamment si les molécules sont fluorescentes, il est possible d'en faire ensuite une image par des techniques connues de l'art antérieur pour prouver le résultat de l'adhésion.

Toutefois, même sans effectuer une mise en contact avec une solution aqueuse, par exemple une solution de protéines, il est possible de prédire après insolation de la brosse que l'effet de l'invention sera obtenu, et ce indépendamment de la réalisation d'un motif réel ultérieur, en mesurant après illumination, s'il existe des creux de profondeur nanométrique dans la brosse aux endroits illuminés au moyen d'un microscope à force atomique (AFM), ou en observant s'il existe des variations de chemin optique dans la brosse optiquement en microscopie à contraste de phase, à ces mêmes endroits. Il est ainsi possible de sélectionner sans autre expérimentation, les brosses de polymère adaptées au procédé de l'invention, notamment comme étant celles pour lesquelles une réduction de la longueur des chaînes de polymère de la brosse est observée après illumination en présence de benzophénone.

Dans un second mode de réalisation de l'invention, le dispositif de la figure 2 est mis en contact avec une solution aqueuse d'une protéine ou une solution aqueuse de nano-billes. Le choix de la nature des protéines ou des nano-billes est effectué parmi les protéines et les nano-billes susceptibles d'adhérer au support afin d'obtenir une image réelle la plus durable possible.

Il est ainsi possible avec le procédé de ce second mode, d'obtenir une image réelle de la zone AB par exemple en utilisant une protéine fluorescente, mais plus généralement un motif d'une protéine sur le substrat anti-salissures pour la protéine qui a été utilisée. De plus, les propriétés sous illumination des substrats anti-salissures permettent de réaliser une fluorescence de valeur continûment variable avec l'éclairement ou la dose de radiation optique reçue par la zone AB et plus généralement une concentration de protéines, de nano-billes ou de brins d'ADN, continûment variable avec l'éclairement dans cette zone, même si cette zone correspond à la limite de résolution du système optique d'éclairage, sans recours à des densités de points binaires pour simuler des concentrations variables de protéines.

Il est ainsi possible d'appliquer l'invention à la réalisation de gradients d'adhésion dans une direction de concentration par exemple d'une protéine, de nano-billes ou de brins d'ADN, le long de la surface du substrat ou de la couche anti-salissures, en alignant bout à bout plusieurs zones de type AB, et en faisant varier l'énergie surfacique délivrée à ces zones, par exemple en les éclairant avec des zones surfaciques (en J/m2) variables, lors de l'étape d'éclairement de la brosse de polymère en présence de benzophénone ou d'impression de l'image latente ou motif adhésif.

Par exemple, un effet adhésif continûment variable pour des protéines, a été obtenu par illumination à dose variable en présence de benzophénone sur une brosse de PEG, pour une diminution d'épaisseur comprise entre 0nm de réduction (pas d'adhésion ou zone hors-motif) et 2nm de réduction (adhésion maximale) pour des brosses de polymères de PEG d'épaisseur estimée à 5nm en dehors des zones d'adhésion.

Dans les modes de réalisation présentés, une gamme de concentration en millimoles de benzophénone par litre de solution aqueuse (mmol/l) de 5mmol/l à 50mmol/l, a été utilisée.

L'invention est susceptible d'application industrielle dans le domaine de la production de substrat pour l'impression de motifs adhésifs d'une protéine sur une brosse de polymère.

## Revendications

1. Procédé pour imprimer un motif adhésif, sur une brosse de polymère s'étendant à la surface d'un support (1) en une couche (2) nanométrique anti-salissures, le procédé comprenant les étapes suivantes :
- mettre la couche (2) en contact avec une première solution aqueuse (4) contenant une benzophénone,
- puis éclairer la couche, par un rayonnement (3) à une longueur d'onde comprise dans le spectre d'absorption de la benzophénone, selon le motif et selon une énergie surfacique, et rincer pour supprimer le contact entre la couche (2) et la première solution aqueuse (4), créant ainsi un motif adhésif qui est une zone sur la brosse de polymère plus adhésive pour des molécules d'intérêt que la surface complémentaire de la zone sur la brosse de polymère, le motif adhésif étant un motif en creux dans la brosse de polymère non recouvert par les molécules d'intérêt.

2. Procédé selon la revendication 1 dans lequel l'épaisseur de la couche (2) est comprise entre 1 nm et 20 nm.

3. Procédé selon la revendication 1 ou 2 dans lequel la longueur d'onde est choisie entre 300 nm et 400 nm.

4. Procédé selon l'une quelconque des revendications 1 à 3 dans lequel ledit polymère est un polyéthylène glycol (PEG).

5. Procédé selon l'une quelconque des revendications 1 à 3 dans lequel ledit polymère est un poly(N-isopropylacrylamide).

6. Procédé selon l'une quelconque des revendications 1 à 5 dans lequel ledit support (1) est en verre.

7. Procédé selon l'une quelconque des revendications 1 à 5 dans lequel ledit support (1) est en PolyDiMethylSiloxane (PDMS).

8. Procédé selon l'une quelconque des revendications 1 à 7, dans lequel on réalise un motif adhésif présentant un gradient d'adhésion à la surface du support (1), par variation spatiale de l'énergie surfacique de l'éclairage.

9. Procédé selon l'une quelconque des revendications 1 à 8, dans lequel la longueur des chaînes de polymère de la brosse de polymère est réduite à l'endroit du motif adhésif.

10. Procédé selon l'une quelconque des revendications 1 à 9, pour imprimer un motif de molécules d'intérêt sur la brosse du polymère, comprenant l'étape supplémentaire suivante :
- mettre la couche (2) en contact avec une seconde solution aqueuse contenant les molécules d'intérêt.

11. Procédé selon la revendication 10, pour imprimer un motif de molécules d'intérêt sur la brosse du polymère, dans lequel les molécules d'intérêt sont des protéines, des nano-billes, des brins d'ADN, des brins d'ARN ou des bactéries.

12. Procédé selon la revendication 10 ou 11, dans lequel, lors de la mise en contact de la couche (2) avec les molécules d'intérêt, ces dernières sont adsorbées sur la couche (2) au niveau du motif adhésif, l'adsorption des molécules d'intérêt se faisant sans apport d'énergie lumineuse.

## Patentansprüche

1. Verfahren zum Drucken eines Haftmusters auf eine Polymerbürste, die sich auf der Oberfläche eines Trägers (1) in einer schmutzabweisenden Nanometer-Schicht (2) erstreckt, wobei das Verfahren die folgenden Schritte umfasst:
- Inkontaktbringen der Schicht (2) mit einer ersten wässrigen Lösung (4), die ein Benzophenon enthält,
- anschließend Belichten der Schicht mit einer Strahlung (3) bei einer Wellenlänge innerhalb des Absorptionsspektrums des Benzophenons gemäß dem Muster und gemäß einer Oberflächenenergie sowie Spülen, um den Kontakt zwischen der Schicht (2) und der ersten wässrigen Lösung (4) aufzuheben, so dass ein Haftmuster erzeugt wird, das eine Zone auf der Polymerbürste ist, die für Moleküle von Interesse adhäsiver ist als die komplementäre Oberfläche der Zone auf der Polymerbürste, wobei das Haftmuster ein vertieftes Muster in der Polymerbürste ist, das nicht von den Molekülen von Interesse abgedeckt wird.

2. Verfahren nach Anspruch 1, wobei die Dicke der Schicht (2) zwischen 1 nm und 20 nm beträgt.

3. Verfahren nach Anspruch 1 oder 2, wobei die Wellenlänge zwischen 300 nm und 400 nm gewählt wird.

4. Verfahren nach einem der Ansprüche 1 bis 3, wobei das Polymer ein Polyethylenglykol (PEG) ist.

5. Verfahren nach einem der Ansprüche 1 bis 3, wobei das Polymer ein Poly(N-isopropylacrylamid) ist.

6. Verfahren nach einem der Ansprüche 1 bis 5, wobei der Träger (1) aus Glas besteht.

7. Verfahren nach einem der Ansprüche 1 bis 5, wobei der Träger (1) aus PolyDiMethylSiloxan (PDMS) besteht.

8. Verfahren nach einem der Ansprüche 1 bis 7, wobei man durch räumliche Variation der Oberflächenenergie der Belichtung ein Haftmuster, das einen Haftgradienten aufweist, auf der Oberfläche des Trägers (1) herstellt.

9. Verfahren nach einem der Ansprüche 1 bis 8, wobei die Länge der Polymerketten der Polymerbürste an der Stelle des Haftmusters verringert ist.

10. Verfahren nach einem der Ansprüche 1 bis 9 zum Drucken eines Musters von Molekülen von Interesse auf die Bürste des Polymers, umfassend den folgenden zusätzlichen Schritt:
- Inkontaktbringen der Schicht (2) mit einer zweiten wässrigen Lösung, die die Moleküle von Interesse enthält.

11. Verfahren nach Anspruch 10 zum Drucken eines Musters von Molekülen von Interesse auf die Bürste des Polymers, wobei die Moleküle von Interesse Proteine, Nanokügelchen, DNA-Stränge, RNA-Stränge oder Bakterien sind.

12. Verfahren nach Anspruch 10 oder 11, wobei beim Inkontaktbringen der Schicht (2) mit den Molekülen von Interesse diese letzteren auf der Schicht (2) am Haftmuster adsorbiert werden, wobei die Adsorption der Moleküle von Interesse ohne Zufuhr von Lichtenergie erfolgt.

## Claims

1. A process for printing an adhesive pattern on a polymer brush extending at the surface of a support (1) and forming a nanometric anti-fouling layer (2), the process comprising the following steps:
- placing the layer (2) in contact with a first aqueous solution (4) containing a benzophenone,
- then illuminating the layer with a radiation (3) at a wavelength within the absorption spectrum of the benzophenone, according to the pattern and according to a surface energy, and rinsing to eliminate the contact between the layer (2) and the first aqueous solution (4), thereby creating an adhesive pattern which is a zone on the polymer brush that is more adhesive for molecules of interest than the supplementary surface of the zone on the polymer brush, the adhesive pattern being a hollow pattern in the polymer brush and not being covered by the molecules of interest.

2. The process of claim 1, wherein the thickness of the layer (2) is between 1 nm and 20 nm.

3. The process of claim 1 or 2, wherein the wavelength is chosen between 300 nm and 400 nm.

4. The process of any one of claims 1 to 3, wherein said polymer is a polyethylene glycol (PEG).

5. The process of any one of claims 1 to 3, wherein said polymer is a poly(N-isopropylacrylamide).

6. The process of any one of claims 1 to 5, wherein said support (1) is made of glass.

7. The process of any one of claims 1 to 5, wherein said support (1) is made of PolyDiMethylSiloxane (PDMS).

8. The process of any one of claims 1 to 7, wherein an adhesive pattern having an adhesion gradient at the surface of the support (1) is produced by spatial variation of the surface energy of the illumination.

9. The process of any one of claims 1 to 8, wherein the length of the polymer chains of the polymer brush is reduced at the location of the adhesive pattern.

10. The process of any one of claims 1 to 9, for printing a pattern of molecules of interest on the polymer brush, comprising the following additional step:
- placing the layer (2) in contact with a second aqueous solution containing the molecules of interest.

11. The process of claim 10, for printing a pattern of molecules of interest on the polymer brush, wherein the molecules of interest are proteins, nanoshells, DNA strands, RNA strands or bacteria.

12. The process as claimed in claim 10 or 11, wherein, when the layer (2) is brought into contact with the molecules of interest, the molecules of interest are adsorbed on the layer (2) at the adhesive pattern, the adsorption of the molecules of interest taking place without provision of light energy.
